# EUROPEAN PATENT APPLICATION

(11) **EP 2 359 750 A1**
(43) Date of publication of application: **24.08.2011**
(21) Application number: 10153596.1
(22) Date of filing: 15.02.2010
(51) Int. Cl.: A61B 10/00, A61M 39/22, F16K 17/19

(54) **Screening device with valve**

(71) Applicant: Delphi Bioscience B.V., 3925 BV Scherpenzeel Gld. (NL)
(72) Inventor: Wiegerinck, Martinus Antonius Hermanus, 5613 CJ, Eindhoven (NL); Coelingh Bennink, Herman Jan Tijmen, 3985 MK Werkhoven (NL)
(74) Representative: Ketelaars, Maarten F.J.M.

(57) **Abstract**

Screening device for delivery of a substance to a body cavity and subsequently retrieving a sample from the body cavity. The screening device (10) has a tubular body (1) provided with one or more openings (8) in a distal end of the tubular body (1), and a container part (14) in communication with the one or more openings (8). Furthermore, the screening device (10) is further provided with a valve assembly (10) positioned between the container part (14) and the one or more openings (8), the valve assembly (20) comprising a first valve part (16a, 16b) to allow a flow of an amount of the substance to pass from the container part (14) to the one or more openings (8), and a second valve part (17) to allow a flow of the sample to pass from the one or more openings (8) to the container part (14).

## Description

### Field of the invention

The present invention relates to a screening device for delivery of a substance to a body cavity and subsequently retrieving a sample from the body cavity, comprising a tubular body provided with one or more openings in a distal end of the tubular body, and a container part in communication with the one or more openings.

### Prior art

International patent application WO2006/033569 discloses such a screening device in various embodiments wherein a plunger is used to expel fluid from a container space inside the tubular body itself.

European patent application EP-A-0 761 246 discloses an apparatus for applying a medical fluid into body cavities. It comprises a pump device, a distribution body and a connecting tube between the pump device and the distribution body. The diameter of distribution body and connecting tube is different.

International patent application WO2006/016869 discloses a delivery system comprising a tubular body with a plunger to expel an emulsion through slits in a distal end of the tubular body. The emulsion is stored inside the tubular body next to the slits.

### Summary of the invention

The present invention seeks to provide an improved screening device, which improves manufacturing efficiency and handling by users of the screening device.

According to the present invention, a screening device as defined in the preamble above is provided, which further comprising a valve assembly positioned between the container part and the one or more openings, the valve assembly comprising a first valve part to allow a flow of an amount of the substance to pass from the container part to the one or more openings, and a second valve part to allow a flow of the sample to pass from the one or more openings to the container part. By providing the valve assembly an easy to use screening device is provided, which is suitable for home use, without any professional help or supervision.

In an embodiment, the first valve part comprises a (bicycle) tire valve type of valve, allowing flow of the substance in a first direction towards the one or more openings when a pressure difference in the first direction over the first valve part exceeds a first threshold value. Such a type of valve provides sufficient resistance against leakage of the fluid from the screening device before use, yet also allows simple and easy operation of the screening device.

In a further embodiment, the second valve part comprises an umbrella type of valve, opening in a second direction from the one or more openings towards the container part, opposite to the first direction, when a pressure difference in the second direction over the second valve part exceeds a second threshold value, the second threshold value being lower than the first threshold value. This allows to recollect the fluid including cells or other biological material from the body cavity easily, by using only small forces such as e.g. provided by a spring or other resilient member.

The tube comprises a moveable plunger in a further embodiment which determines the volume of the container part. By moving the plunger towards the one or more openings, fluid is effectively expelled, and by movement of the plunger in the opposite direction, the sample is recollected inside the screening device.

In a further embodiment, the container part comprises a capsule containing the substance. A capsule may provide better resistance against leakage or evaporation of the fluid by choosing the right material. The capsule may be ruptured by applying a force onto it to release the fluid. In an even further embodiment, the container part comprises a capsule punching element, the capsule punching element breaking the capsule when the volume of the container part is diminished. As a result of this element, less force is needed to actually break the capsule.

The moveable plunger is connected (mechanically) to an operating part assembly, which allows easy operation of the screening device, even using only one hand. The operating part assembly is provided with a grip part having a central opening in a further embodiment, allowing e.g. to hold the screening device using one's thumb. The grip part may also be used to indicate to which depth the screening device is to be brought into the body cavity for proper use.

In a further embodiment, the screening device comprises a blocking assembly which allows only a one-time operation of the operating part assembly.

The screening device comprises a further container part in an even further embodiment for accommodating a preservative before use. The preservative is mixed with the recollected sample during use, and allows safe transportation back to a laboratory.

The further container part is defined by a plunger-valve element in the container part in a further embodiment, the plunger-valve element comprising a plunger part and a valve part, allowing flow only from the further container part to the container part. In an alternative embodiment, the further container part is defined by a plunger-valve element in the container part, the plunger-valve element comprising a plunger part and a valve part, allowing flow only from the container part to the further container part.

In a further aspect, the present invention relates to a valve assembly, which may be used in a screening device. The valve assembly (or two-way valve) comprises a first valve part to allow a flow of an amount of the substance to pass in a first direction, and a second valve part to allow a single flow of a sample to pass in a second direction opposite to the first direction. In a further embodiment the first valve part comprises a tire valve type of valve, allowing flow of the substance in the first direction when a pressure difference in the first direction over the first valve part exceeds a first threshold value. In an even further embodiment, the second valve part comprises an umbrella type of valve, opening in the second direction when a pressure difference in the second direction over the second valve part exceeds a second threshold value, the second threshold value being lower than the first threshold value. These valve assemblies may be advantageously used in a screening device used to expel fluid in a body cavity and recollect a sample form that body cavity, but may also be applied in other applications.

### Short description of drawings

The present invention will be discussed in more detail below, using a number of exemplary embodiments, with reference to the attached drawings, in which
Fig. 1 shows a side view of a screening device according to an embodiment of the present invention;
Fig. 2 shows a cross sectional view of the screening device of Fig. 1;
Fig. 3 shows a cross sectional view of a valve assembly as used in the screening device of Fig. 1;
Fig. 4 shows a side view of a further embodiment of the screening device;
Fig. 5 shows a cross sectional view of the screening device of Fig. 4; and
Fig. 6 shows a three-dimensional view of the operating knob of the screening device of Fig. 4.

### Detailed description of exemplary embodiments

The present invention relates to a screening device or sampler 10 which can be used for diagnostics, e.g. in screening programs involving collection of cells from the cervix and/or uterus in a large group of people. The present screening device 10 is particularly useful in microbiological diagnostics, such as bacterial or viral detection, molecular diagnostics, cytology etc.

In general, the screening device 10 is used to deliver a fluid or substance (e.g. a gel or aqueous solution) in a body cavity, and to subsequently retrieve a sample comprising the fluid with cells or other biological material suspended therein. More general, the screening device 10 can be used in any body cavity, e.g. a mucosal cavity, such as a vaginal cavity, rectum, throat etc.

The screening device 10 is intended for single use only, and is especially suitable for home use without any medical supervision. After use of the screening sampler 10, it can be sent to a laboratory for further testing in a laboratory environment of the sample material obtained.

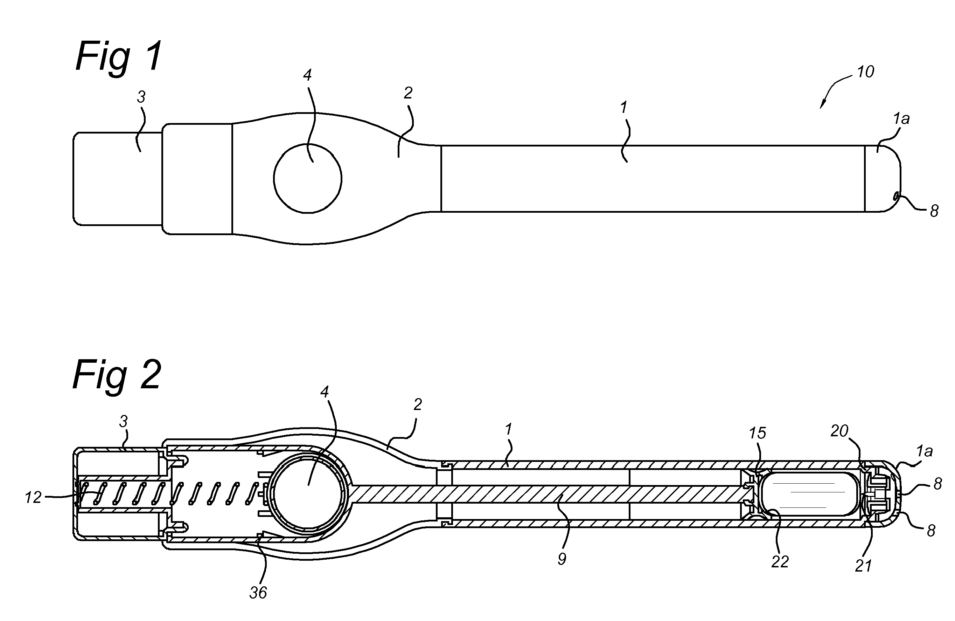

A first general embodiment of the screening device 10 according to the present invention is shown in a side view in Fig. 1. The screening device 10 comprises a tubular body 1 having one or more openings 8 in a distal end 1a of the tubular body 1. The distal end 1a may be a separate element, attached to the tube 1, or may be an integral part of the tube 1. The openings 8 are being used both to deliver a fluid in the body cavity, and to collect fluid (with cells) from the body cavity. The screening device 10 further comprises an operating part assembly, comprising a grip part 2 and an operating knob 3. In this embodiment, the grip part 2 is attached to the tube 1 at a position indicating how far a user needs to bring in the tube 1 in the body cavity (e.g. by using different colors or materials for the tube 1 and the grip part 2).

The grip part 2 in this embodiment, is provided with an opening 4. This allows a user to firmly grasp the screening device 10 e.g. using the thumb, and to operate the operating knob 3 with one or more fingers of the same hand.

A cross sectional view of the embodiment of Fig. 1 is shown schematically in Fig. 2. Near to the one or more openings 8, in this embodiment a valve assembly 20 is positioned between a container part 14 of the tube 1 and the openings 8. In the ready-to-use form of the screening device 10, the container part 14 comprises the fluid, and after use, the collected fluid including cells or other biological material from the body cavity.

In the embodiment shown, the fluid is contained in a capsule 22, e.g. using a plastic material, to prevent loss of the fluid before use (e.g. by evaporation). During operation, the capsule 22 may be punctured using a capsule puncturing element 21. In the embodiment shown, the capsule puncturing element 21 is provided on a part of the valve assembly 20 directed towards the capsule 22. More in general, the capsule puncturing element 21 is provided in a manner that when the volume of the container part 14 is diminished, the capsule 22 is broken, allowing the fluid to flow through the openings 8.

In a further embodiment, no capsule puncturing element 21 is provided, and the capsule 22 is ruptured when applying pressure to it, by choosing the right material, or by providing weak spots in the material of the capsule 22.

The capsule 22 allows a more safe containment of the fluid before actual use, preventing evaporation of the fluid, and preventing contamination before use. The capsule 22 may be provided in a screening device 10 with or without other elements discussed in the various embodiment, i.e. a screening device may be provided for delivery of a substance to a body cavity and subsequently retrieving a sample from the body cavity, comprising a tubular body 1 provided with one or more openings 8 in a distal end 1a of the tubular body 1, and a container part 14 in communication with the one or more openings 8, wherein the container part 14 comprises a capsule 22 containing the substance.

In a further embodiment, the fluid is contained in the container part 14, the borders of which are formed by an inner wall of the tube 1, a plunger 15 and the valve assembly 20. The plunger 15 is moveable within the tube 1 and defines the volume of the container part 14.

The plunger 15 is connected to a connecting element 9, which is fixedly connected to the knob 3 of the operating part assembly. In this embodiment, the form of the connecting element 9 is such as to allow the opening 4 to be present in the grip part 2. Furthermore, the connecting element 9 is shaped to be able to move in two opposite directions in the grip part 2 (e.g. using a co-cylindrical form as shown in the embodiment of Fig. 2).

A rim 13 is provided inside the grip part 2 (bordering the opening 4), which seats a spring 12 on one side. The other side of the spring 12 is seated in a tube part 11 connected to or part of the knob 3. The spring 12 provides a force on the connecting element 9 (and the plunger 15) trying to enlarge the volume of the container part 14 when no force is exerted on the knob 3. As a result, a user can expel the fluid by applying force on the knob 3, and collect fluid and specimen from the body cavity by simply releasing the knob 3.

In an embodiment, the connecting element 9 is provided with locking means 35, which allow the connecting element 9 to (temporarily) stay in a position where the container part 14 has the smallest volume (fluid expelled). After again pressing the knob 3, the locking means 35 are again released, allowing the spring 12 to exert force and collect the sample in the container part 14. The locking means 35 can be implemented using a ball point operation mechanism (known as such), or other temporary arresting means.

The outside surface of the tubular body 1 is provided with a shallow texture in a further embodiment, which aids in a smooth insertion of the screening device 10 in the body cavity.

The valve assembly 20, or two-way valve, is positioned between the container part 14 and the one or more openings 8. The valve assembly 20 comprises a first valve part 16a, 16b to allow a single flow of an amount of the fluid to pass from the container part 14 to the one or more openings 8, and a second valve part 17 to allow a single flow of the sample taken to pass from the one or more openings 8 to the container part 14.

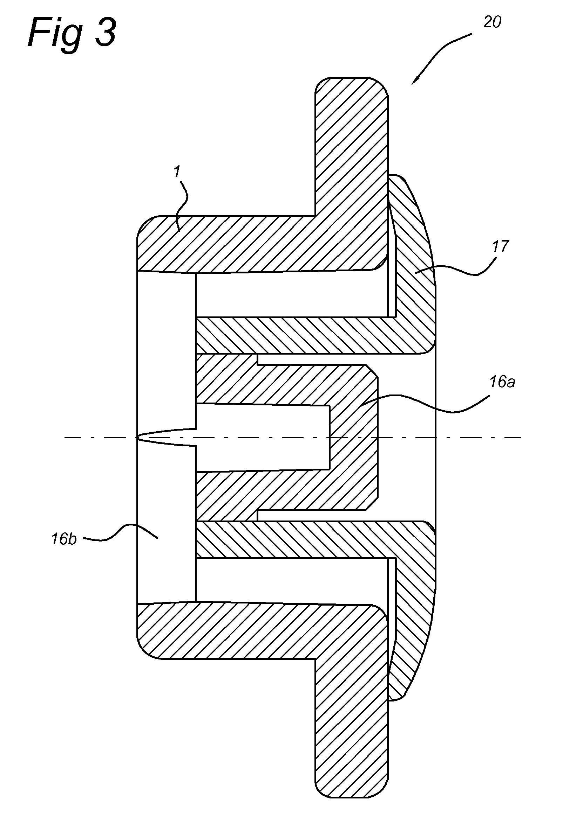

In Fig. 3 a cross sectional view in detail is shown of the valve assembly 20 as used in the screening device 10 of Fig. 1 and 2. A part of the tube 1 is shown, in which the valve assembly 20 is positioned. The first valve part 16a, 16b comprises a cylindrical stiff part 16a, resting against a disc shaped part 16b. Furthermore, the stiff part 16a rests against the inside of the second valve part 17, which has an umbrella type of shape and rests against a shoulder of the inner wall of the tube 1.

The first valve part 16a, 16b is a one-way valve and is arranged to act as a tire type of valve, i.e. it allows fluid to pass in a first direction (as indicated by the arrow labeled 'OUT') when applying sufficient pressure, and prevents fluid to pass in the opposite direction (second direction, labeled as 'IN' in Fig. 3). The choice of materials and structure of the stiff part 16a and disc shaped part 16b make that a first threshold value of pressure must be exceeded before the fluid can flow in the first direction.

The second valve part 17 is also a one-way valve, but is arranged to allow fluid to pass in the second direction ('IN' in Fig. 3). This is accomplished by an umbrella type of form of the second valve part, partly overlapping a rim of the tube 1. As the outer circular part is flexible, a pressure of a second threshold value, which is lower than the first threshold value, already allows fluid to re-enter the container part 14 from the one or more openings 8.

In co-operation with the operating part assembly 2 as described above, this allows to expel fluid from the screening device 10 by applying some force, and to re-collect a sample from the body cavity using the spring force action only.

It is noted that the valve assembly 20 in itself may be used for other applications, i.e. a separate valve assembly may be provided comprising a first valve part 16a, 16b to allow a flow of an amount of the substance to pass in a first direction, and a second valve part 17 to allow a single flow of a sample to pass in a second direction opposite to the first direction. The first valve part 16a, 16b may comprises a tire valve type of valve, allowing flow of the substance in the first direction when a pressure difference in the first direction over the first valve part 16a, 16b exceeds a first threshold value. The second valve part 17 may comprise an umbrella type of valve, opening in the second direction when a pressure difference in the second direction over the second valve part 16a, 16b exceeds a second threshold value, the second threshold value being lower than the first threshold value.

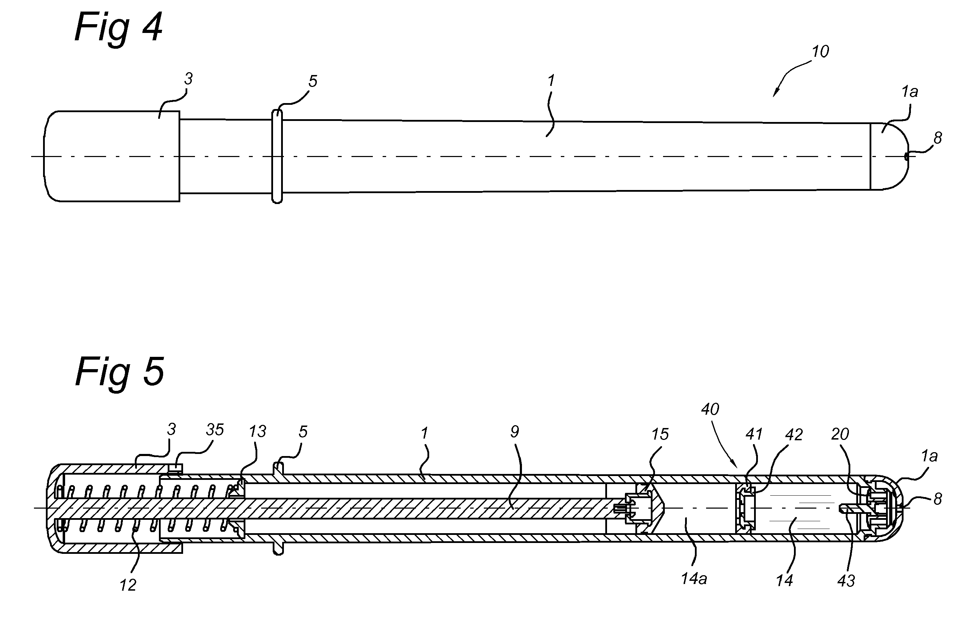

In Fig. 4, a side view of a second embodiment of the screening device 10 is shown. Again, a tube 1 is provided, with a distal end 1a (integral part of tube 1, or separate element) which is provided with one or more openings 8 to implement a spray nozzle allowing efficient expelling of the fluid in a body cavity. In this embodiment, the grip part 2 is omitted, and only a rim 5 is provided as part of the tube 1.

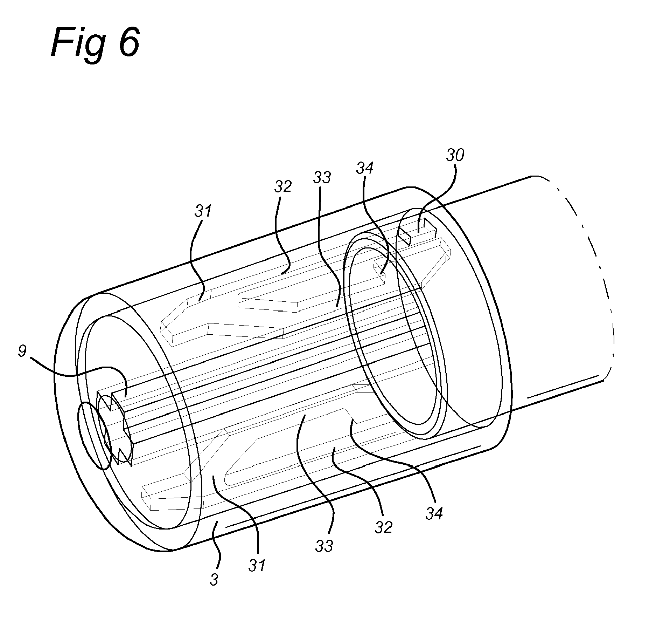

As shown in the cross sectional view of Fig. 5, the knob 3 is provided as a simple extension of the connecting element 9, allowing movement of the plunger 15 inside the tube 1. The rim 5 in co-operation with the knob 3 determines how far the plunger 15 can be moved. In the tube 1, a ring 13 is provided resting against a thinner wall part of the tube 1. A spring 12 is provided between the knob 3 and the ring 13, allowing a similar operation as the embodiment described above. The knob 3 is furthermore provided with an opening 36 at the edge of the knob 3, the function of which will be explained below with reference to Fig. 6.

On the side of the tube 1 where the one or more openings 8 are located, a valve assembly 20 is provided, very similar to the valve assembly 20 as described above. This valve assembly 20 allows fluid to be expelled from the openings 8 when applying a force on the knob 3, and fluid including cells or other biological material to be collected again in container part 14.

In this embodiment, a plunger-valve element 40 is provided, which defines the container part 14 (with the fluid before use), and at the same time a further container part 14a, which before use contains a preservative.

The plunger-valve element 40 comprises a plunger part 41 and a valve part 42, the valve part 42 allowing only flow of the preservative from the further container part 14a to the container part 14. In operation, the plunger 15 is moved towards the distal end 1a by applying a force on knob 3 (via connector element 9)This forces the preservative from the further container part 14a into the container part 14, which in its turn results in that the fluid is ejected from the one or more openings 8. The dimensions of the tube 1 and connector element 9, in combination with the dimension of the knob 3 and position of rim 5, assure that sufficient volume of the fluid is ejected from the one or more openings 8.

When at this point the knob 3 is released, the spring 12 forces the plunger 15 back in the tube 1 to its original position, creating an under pressure in the container part 14 and further container part 14a. Fluid with collected cells or other biological material is collected via the one or more openings 8 into the container part 14, where it is mixed with the preservative. As the valve part 42 in combination with the plunger part 41 allows fluid only to pass in the direction of the one or more openings, the reverse movement of the plunger 15 will also result in a shift of the plunger-valve element 40 away from the one or more openings, as a result of which the volume of the container part 14 increases to accommodate the mixture of fluid, preservative and collected sample material.

The plunger-valve element 40 may also be arranged to allow fluid flow from the container part 14 to the further container part 14a only. When applying force to the operating knob 3, the plunger valve element 40 blocks, as a result of which it moves in the direction of the one or more openings 8, thereby reducing the volume of the container part 14. After expelling the fluid, the plunger-valve element has moved towards the one or more openings 8. When releasing the operating knob 3, the plunger-valve element 40 is kept in position, however, fluid with collected cell or biological material is allowed to flow into the further container part 14a, and to mix with the preservative.

This embodiment is advantageous for easily retrieving the sample after use, e.g. in a laboratory: a second application of force on the operating knob 3 (which may be accomplished by breaking a blocking element) again advances the plunger-valve element 40 in the direction of the one or more openings 8. A protrusion provided on the valve assembly 20 pushes the valve part 41 out of the plunger part 42, thereby providing a pathway for (partly) expelling the sample from the further container part 14a. A further application of force on the operating knob 3 holding the screening device 10 in a vertical orientation allows to expel the rest of the sample.

The elements which form the container part 14 with fluid and further container part 14a with preservative may also be applied to the embodiment shown and described by reference to Fig. 1 and 2. More general, a screening device may be provided for delivery of a substance to a body cavity and subsequently retrieving a sample from the body cavity, comprising a tubular body 1 provided with one or more openings 8 in a distal end of the tubular body 1, and a container part 14 in communication with the one or more openings 8, and a further container part 14 in communication with the container part for accommodating a preservative before use. The further container part 14a may be defined by the plunger-valve element 40 as described above.

In Fig. 6, a three dimensional view is shown with ghost lines showing the inner side of the knob 3 as positioned on the connecting element 9. In this embodiment, the end side of the tube 1 (opposite the distal end 1a in Fig. 4 and 5) is provided with a cam element 30, which co-operates with a guiding profile 31 on the inner surface of knob 3. The guiding profile 31 is formed to allow a one-time single operation of the screening device 10, by having two pathways 32, 33. From the start position as indicated in Fig. 6 (not yet used), the cam follows the first pathway 31, at the end slightly rotating the knob 3. When the knob 3 is released, the cam follows the second pathway 33, and during the release the knob 3 is rotated further and back, until the cam 30 is in its end position at the end of the second pathway 33. The second pathway 33 is formed with a blocking surface 34, with the result that the knob 3 cannot be actuated again. In a further embodiment, the cam element 30 and guiding profile 31 may be provided double, in order to provide a more stable operating mechanism.

By the slight rotation of the knob 3 from the initial position of the cam 30 in the first pathway 32 to the final position in second pathway 33, the opening 32 is shifted over the surface of the tube 1, and e.g. exposes an indicator on the outside surface of the tube 1. E.g. a small colored surface may act as indicator that the screening device 10 has been used.

An operating and indicator implementation (elements 30-34) as shown in the embodiment of Fig. 6 may be similarly applied to the embodiment of the screening device 10 as shown and discussed with reference to Fig. 1 and 2.

Likewise, in the embodiment of the screening device as shown in Fig. 4 and 5, the fluid and/or the preservative may be held (before operation) in a capsule 22 as described with reference to the embodiment of Fig. 2 above. A further puncturing element 21 or further puncturing elements 21 may be provided, or alternatively, the capsule(s) 22 may be ruptured by applying pressure.

In the embodiments described above, the distal end 1a may be provided with a removable seal (not shown) covering the one or more openings 8 before use. This is beneficial in preventing evaporation of the fluid in the screening device 10 and also provide an increased hygienic level.

The screening device 10 can be put in a package after use for sending it to a laboratory for further analysis of the sample. The package may be provided with special features to allow secure transport of the sample (e.g. to follow requirements in some countries for sending biological material by mail). These features may include wrapping in a sufficiently large amount of absorbent material to ensure that even in case of leakage or breaking of the screening device 10, no biological material is able to exit the package. The invention may therefore also be embodied as a kit comprising a screening device 10 according to one of the embodiments described herein and a re-usable package. The re-usable package may be a re-closable package comprising absorbent material, and can be used to ship the screening device 10 to each person in a screening group, and to resend the screening device 10 to a laboratory for further processing.

Furthermore, the screening device 10 may also be delivered to the user in a special package, including a separate container for the fluid, enabling the user to fill the screening device 10 with fluid just before use. After use, the sample may then be transferred from the screening device 10 to a transport container (e.g. as part of the package described above).

It is noted that it is also possible to use the present screening device 10 for therapeutic purposes, i.e. to deliver a substance from the container part 14 to the body cavity. This may be achieved by activating the screening device 10 by pressing the operating knob 3 once the screening device 10 is in the body cavity, and retrieving the screening device 10 from the body cavity while keeping pressure on the operating knob 3 14 to prevent substance from entering the screening device 10.

The screening device 10 according to the various embodiments described above are easy to manufacture. As only very little parts are needed for the entire screening device 10 with the improved functionality as described, it is also possible to manufacture the screening device 10 cost effectively. In a further embodiment, the tubular body 1 has a lightly tapered shape, in which a diameter at a proximal end is larger than a diameter at the distal end (e.g. a tapering of 1-2°). The tapered shape allows casting the tubular body 1 from a plastic material, e.g. using casting or injection molding techniques.

The above described alternatives of the screening device 10 are exemplary embodiments only. Further modifications of and alternatives for the various elements of the screening 10 may be envisaged by the skilled person and are considered to be within the scope of the present invention, which is defined in the accompanying claims.

## Claims

1. Screening device for delivery of a substance to a body cavity and subsequently retrieving a sample from the body cavity, comprising
a tubular body (1) provided with one or more openings (8) in a distal end of the tubular body (1), and a container part (14) in communication with the one or more openings (8), the screening device (10) further comprising a valve assembly (10) positioned between the container part (14) and the one or more openings (8), the valve assembly (20) comprising a first valve part (16a, 16b) to allow a flow of an amount of the substance to pass from the container part (14) to the one or more openings (8), and a second valve part (17) to allow a flow of the sample to pass from the one or more openings (8) to the container part (14).

2. Screening device according to claim 1, wherein the first valve part (16a, 16b) comprises a tire valve type of valve, allowing flow of the substance in a first direction towards the one or more openings (8) when a pressure difference in the first direction over the first valve part (16a, 16b) exceeds a first threshold value.

3. Screening device according to claim 2, wherein the second valve part (17) comprises an umbrella type of valve, opening in a second direction from the one or more openings (8) towards the container part (14), opposite to the first direction, when a pressure difference in the second direction over the second valve part (16a, 16b) exceeds a second threshold value, the second threshold value being lower than the first threshold value.

4. Screening device according to any one of claim 1-3, wherein the tube (1) comprises a moveable plunger (15) which determines the volume of the container part (14).

5. Screening device according to any one of claim 1-4, wherein the container part (14) comprises a capsule (22) containing the substance.

6. Screening device according to claim 5, wherein the container part (14) comprises a capsule punching element, the capsule punching element breaking the capsule (22) when the volume of the container part (14) is diminished.

7. Screening device according to any one of claim 1-6, wherein the moveable plunger (15) is connected to an operating part assembly (2, 3)

8. Screening device according to claim 7, wherein the operating part assembly is provided with a grip part (2) having a central opening (4).

9. Screening device according to claim 7 or 8, wherein the screening device comprises a blocking assembly (30-34; 35), the blocking assembly (30-34; 35) allowing only a one-time operation of an operating assembly (2, 3).

10. Screening device according to any one of claim 1-9, further comprising a further container part (14a) for accommodating a preservative before use.

11. Screening device according to claim 10, wherein the further container part (14a) is defined by a plunger-valve element (40) in the container part, the plunger-valve element (40) comprising a plunger part (41) and a valve part (42), allowing flow only from the further container part (14a) to the container part (14).

12. Screening device according to claim 10, wherein the further container part (14a) is defined by a plunger-valve element (40) in the container part (14), the plunger-valve element (40) comprising a plunger part (41) and a valve part (42), allowing flow only from the container part (14) to the further container part (14).

13. Valve assembly (20) comprising a first valve part (16a, 16b) to allow a flow of an amount of the substance to pass in a first direction, and a second valve part (17) to allow a single flow of a sample to pass in a second direction opposite to the first direction.

14. Valve assembly according to claim 13, wherein the first valve part (16a, 16b) comprises a tire valve type of valve, allowing flow of the substance in the first direction when a pressure difference in the first direction over the first valve part (16a, 16b) exceeds a first threshold value.

15. Valve assembly according to claim 13 or 14, wherein the second valve part (17) comprises an umbrella type of valve, opening in the second direction when a pressure difference in the second direction over the second valve part (16a, 16b) exceeds a second threshold value, the second threshold value being lower than the first threshold value.
